# EUROPEAN PATENT SPECIFICATION

(11) **EP 0 837 947 B1**
(45) Date of publication and mention of the grant of the patent: **17.12.2003**
(21) Application number: 96920986.5
(22) Date of filing: 27.06.1996
(51) Int. Cl.: C12Q 1/02, G01N 33/18

(54) **TOXICITY DETECTION**
TOXIZITÄTSNACHWEIS
DETECTION DE TOXICITE

(30) Priority: 28.06.1995 GB 9513123
(43) Date of publication of application: 29.04.1998
(73) Proprietor: SEVERN TRENT WATER LIMITED, Birmingham, B26 3PU (GB)
(72) Inventor: UPTON, John, Edmund, Leicestershire, LE12 5TB (GB); PICKIN, Stephen, Roy, Woodburn Green Buckinghamshire HP10 0HG (GB)
(74) Representative: Makovski, Priscilla Mary
(86) International application number: GB9601539
(87) International publication number: WO97001643

(56) References cited:
- EP-A- 0 320 483
- EP-A- 0 486 443
- SE-A- 9 100 926
- DATABASE WPI Section Ch, Week 9344 Derwent Publications Ltd., London, GB; Class D04, AN 93-349028 XP002000522 & SE,A,9 200 352 (ANOX AB) , 8 August 1993
- JOURNAL OF CHEMICAL TECHNOLOGY AND BIOTECHNOLOGY, vol. 59, no. 4, April 1994, pages 321-333, XP000440851 P. A. VANROLLEGHEM ET AL.: "An on-line respirographic biosensor for the characterization of load and toxicity of wastewaters."
- WATER RESEARCH, vol. 28, no. 9, 28 September 1994, pages 2029-2031, XP000451467 E. ARVIN ET AL. : "A mini-nitrification test for toxicity screening, MINNTOX."

## Description

This invention relates to the detection of toxic substances, and finds application in the detection of substances toxic to bacteria in waste water. The invention relates particularly, but not exclusively, to the detection of substances toxic to nitrifying, denitrifying and carbon-degrading bacteria.

Various legislation exists which seeks to control the amount of harmful materials present in waste water for example limiting the levels of ammonia which can be discharged to water courses. These have been introduced to protect water courses from the harmful environmental effects of ammonia.

A widely used method of controlling ammonia levels in water is the biological oxidation of ammonia to nitrate using nitrifying bacteria. However, these bacteria are very sensitive to, and are easily inhibited by, a wide range of toxic substances, for example organic compounds and heavy metals. Similarly, other bacteria types are used to control the levels of other harmful materials in waste water. These also are susceptible to toxic substances.

Waste water carrying such substances can have devastating effects if allowed to enter waste water treatment plants, where bacteria are often used. There is therefore a need for a method of detecting these toxic substances prior to the waste water entering the plant.

This may be achieved by assessing the effect of such substances on bacteria, for example the inhibition of bacteria may be measured.

various methods for assessing the inhibition of nitrifying bacteria already exist. For example batch flask tests using liquid cultures are often used. However, a wide range of sources of nitrifying bacteria are employed in these, and this, and the inherent variability of such tests, makes them difficult to standardise and reproduce.

Fixed film detectors have also been proposed. In these, nitrifying cultures are grown on fixed, solid media. However, a number of problems exist with these detectors. For example, the media used can take a long time to be sufficiently colonised to achieve the high number of bacteria and therefore the high rate of nitrification required for an effective detector. This start-up time could be as long as eight weeks. Should the bacteria be killed off by a toxic shock, requiring replacement of the media, an eight-week down-time would ensue before the detector became operational again. In addition, even if sufficient nitrifying bacteria are colonised within such media, they do not adhere well to these media. It is therefore difficult to retain high numbers of bacteria in the detector, which limits detector effectiveness and response time. Of particular importance is the fact that the culture which develops on a medium can not be fully standardised; each detector may grow a slightly different culture. Results from such detectors are therefore difficult to standardise and reproducibility is poor.

SE-A-9200352 discloses an arrangement by means of which it is possible to detect substances occurring in effluents which are toxic for nitrifying microflora in biological effluent treatment works where a partial quantity of the effluents to be treated is separated off and taken to a test reactor forming part of the arrangement which is located upstream of the treatment works. The test reactor contains nitrification bacteria immobilised in balls of alginate or carrageen.

EP-A-320483 discloses a method of encapsulating biological material such as microbes and proteins in a polymer matrix to enhance the use of the materials as agricultural agents (e.g. herbicides, insecticides).

The invention provides, in one of its aspects, a method of testing a fluid for the presence of substances which are toxic to selected bacteria in which bacteria held immobilised by encapsulation in a polyvinyl alcohol medium are exposed to the fluid and the effects of the fluid on one or more bacteriological actions selected from biological nitrification, denitrification, and phosphate uptake and release are assessed, wherein the bacteria have been immobilised by encapsulation using a freeze/thaw technique.

Various bacteriological actions may be assessed, for example biological nitrification, denitrification, and phosphate uptake and release.

The encapsulation technique should preferably allow rapid recovery of bacteria activity after encapsulation. The technique should also preferably allow nutrients and gases, for example oxygen, to reach the bacteria. The freeze/thaw encapsulation technique is used. This method enables the production of large volumes of encapsulated bacteria.

Polyvinyl alcohol is used as the medium for encapsulation for several reasons. It is non toxic to at least nitrifying bacteria. It has good structural integrity, and resists breaking up e.g. when fluidised by air. Polyvinyl alcohol gives a polymer medium which is non ionic, and does not therefore selectively absorb compounds onto its surface. In addition, polyvinyl alcohol is a bulk commodity, which is readily available and low in cost.

The bacteria are preferably distributed amongst many small pieces of the polyvinyl alcohol medium, e.g. small cubes or spheres. Preferably, these have a long shelf-life and can be easily stored.

Using such a method to test for toxic substances has several advantages. Encapsulation allows complete specificity of the type of bacteria held within the medium, ensuring a standard reaction for all detectors, which is reproducible. It also allows a high number of bacteria to be quickly captured and retained in the medium, giving effective detectors with good response times. Depending on the encapsulation technique used, the time taken for the bacteria to become fully active may be short, giving detectors with good start-up times. Using a suitable form of the polyvinyl alcohol medium makes the immobilised bacteria easy to store. Bacteria in a detector can therefore be quickly replaced if necessary, thereby restoring detector function in a short period of time.

Various conditions in the fluid essential to the well-being of the bacteria (e.g. temperature, pH level and oxygen level) may be monitored.

The invention provides, in another of its aspects, a method of constructing a detector for use in testing a fluid for the presence of substances which are toxic to selected bacteria, comprising immobilising bacteria by encapsulation in a polyvinyl alcohol medium and introducing the encapsulated bacteria into a detector vessel whereby the effects of the fluid on them can be assessed, wherein the bacteria have been immobilised by encapsulation using a freeze/thaw technique.

The method may further comprise monitoring one or more actions of the bacteria after introduction into the detector vessel until such action or actions reaches a threshold value.

According to a third aspect of the invention, there is provided a detector for the detection of substances which are toxic to selected bacteria comprising a detector vessel in which bacteria immobilised by encapsulation in a polyvinyl alcohol medium are situated and into which a fluid to be tested can be introduced, and means for assessing effects of the fluid on one or more bacteriological actions selected from biological nitrification, denitrification, and phosphate uptake and release wherein the bacteria have been immobilised by encapsulation using a freeze/thaw technique.

The means for assessing effects of the fluid on one or more actions of the bacteria may comprise means to which substances indicative of action of the bacteria are fed from the detector vessel. Alternatively, the means for assessing the effects of the fluid may comprise means arranged to assess substances within the detector vessel which are indicative of action of the bacteria.

The detector vessel may comprise an outlet through which substances indicative of action of the bacteria can leave the detector. Such substance or substances may each be fed to an appropriate monitor, where the action or actions of the bacteria are assessed. This information can then be used to determine if any toxic substances are present in the fluid under test. Additionally or alternatively a monitor or monitors may be provided within the detector vessel which may assess the action or actions of the bacteria.

The detector vessel may be of any suitable shape, for example a tubular body. It may be manufactured from any suitable material, for example plastic, and in a preferred form is made from transparent plastic.

The encapsulated bacteria may take any suitable form, and are preferably distributed amongst many small pieces of the medium e.g. small cubes or spheres. These may be immersed in a liquid within the detector, which liquid may be water. The encapsulated bacteria preferably occupy approximately 50% of the volume of the detector vessel.

The detector may further comprise means for monitoring conditions essential to the well-being of the bacteria within the detector vessel. This may take any suitable form. For example, monitoring means may be provided within the detector vessel, or substances indicative of the conditions fed to external monitoring means. Various conditions within the detector may be monitored. For example, the pH level, the temperature or the oxygen level may be measured. The conditions within the detector which require monitoring will depend on the type of bacteria used.

The detector may further comprise means for controlling the conditions within the detector vessel, for example, a heater may be provided to maintain the temperature at a suitable level, or a pH controller may be provided. The levels of various gases may be controlled depending on the conditions required by the bacteria. For example, for bacteria which require aerobic conditions, means for introducing oxygen e.g. an air compressor and air stone may be provided in the detector vessel to maintain a suitable level of dissolved oxygen therein.

The output of any monitor may be fed to any suitable data processor, which may display this information in any suitable form, for example, visually on a screen. The data processor may also determine other data, for example the percentage inhibition of the bacteria.

The output of the monitor or monitors may alternatively or in addition to the above, be used to generate a warning which may for example be an audio or visual warning.

The detector thus provided is effective yet simple to manufacture and install. It is easy to replace and has no lengthy start-up or down-time as in other detectors. Unlike flask tests and fixed film detectors, standard and reproducible results may be obtained.

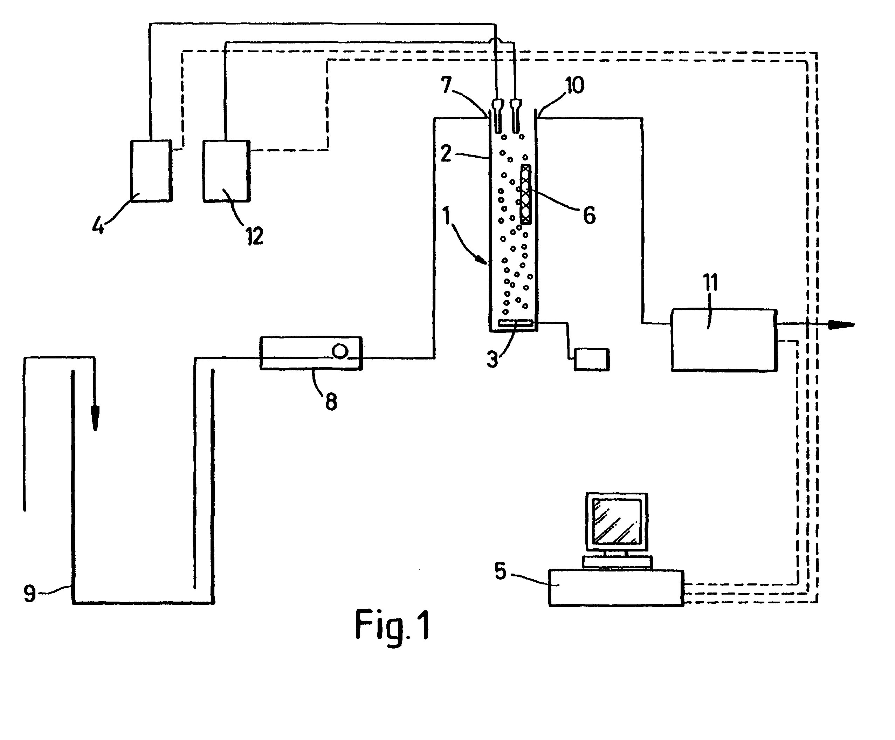

There now follows a description, to be read with reference to the accompanying drawing, of a detector which illustrates the invention by way of example. The drawing is a schematic representation of the detector and associated apparatus.

Nitrifying bacteria are widely present in waste water treatment plants and it is important to be able to determine if water intended for discharge to such plants contains any toxic substances harmful to these bacteria.

A detector 1 comprises a 1.5 litre clear plastic tubular body forming a detector vessel 2. This contains **Nitrosomonas** and **Nitrobacter** nitrifying bacteria encapsulated in 2-3mm cubes of polyvinyl alcohol, the cubes being immersed in water. An air compressor pumps air to an air stone 3 situated within the detector. This is used to fluidise the cubes and maintain the dissolved oxygen level above 5.0 mg/l. A dissolved oxygen monitor 4 is also provided, information from which is relayed to a data processor 5, and used to determine the oxygen conditions within the detector vessel 2. A heater 6 is also provided within the detector, to maintain the temperature of the water and encapsulated bacteria at a suitable level.

An inlet 7 is provided on the detector 1. This is connected to a feed pump 8 which is arranged to draw from a feed tank 9. An outlet 10 is also provided on the detector 1. This is connected to an ammonia monitor 11. A pH monitor 12 is also provided. The outputs of the ammonia monitor and the pH monitor are relayed to the data processor 5.

A waste water sample which requires testing is passed into the feed tank 9 and fed by the pump 8 via the inlet 7 into the detector body 2. The ammonia level discharged through the outlet 10 is measured by the ammonia monitor 11. The output from this monitor is relayed to the data processor 5, where it is used to calculate the percentage inhibition of the nitrifying bacteria, and hence determine if any toxic substances are present in the water sample.

The detector is manufactured as follows. A suitable culture of nitrifying bacteria can be obtained commercially. The nitrifying bacteria are then immobilised by encapsulation in polyvinyl alcohol as follows, using a known freeze thaw technique.

One litre of fully settled nitrifying bacteria is filtered overnight with a Whatman No. 1 filter paper and funnel, forming a paste on the filter paper. A 20% (weight/volume) aqueous solution of polyvinyl alcohol, of a suitable grade, is added to tap water. The paste is added to the polyvinyl alcohol and thoroughly mixed. The resultant mixture is then poured into a flat tray and frozen at-20°C for 24 hours. The resultant water-insoluble sheet of bacteria in polyvinyl alcohol is then allowed to thaw for 24 hours and cut into 2-3mm cubes. The cubes are washed with tapwater for 24 hours to remove any surplus polyvinyl alcohol.

It will be appreciated that a large number of cubes may be manufactured in this way. These may be easily stored at 4°C, and have a shelf-life of between 8 and 12 months. The cubes may be stored in a cartridge, which can easily be placed in a detector to replace bacteria which have been killed by a toxic shock.

The detector vessel is filled with tap water and cubes of encapsulated bacteria, the cubes occupying approximately 50-55% of the volume. The detector vessel is aerated to fluidise the cubes and maintain the dissolved oxygen level above 5.0 mg/l. The detector is fed ammonium carbonate (at 20.0 mg/l N-NH₃) and potassium dihydrogen ortho phosphate (at 2.0 mg/l P-PO₄). The flow to the detector is increased in gradual increments over a 2 to 3 day period to a point where the residence time is approximately 30 minutes. At this residence time the outlet ammonia level should be below 5.0 mg/l N-NH₃. Once the outlet has stabilised to give an ammonia level below this threshold value, the detector is ready for use.

## Claims

1. A method of testing a fluid for the presence of substances which are toxic to selected bacteria in which bacteria held immobilised by encapsulation in a polyvinyl alcohol medium are exposed to the fluid and the effects of the fluid on one or more bacteriological actions selected from biological nitrification, denitrification, and phosphate uptake and release are assessed, wherein the bacteria have been immobilised by encapsulation using a freeze/thaw technique.

2. A detector (1) for the detection of substances which are toxic to selected bacteria comprising a detector vessel (2) in which bacteria immobilised by encapsulation in a polyvinyl alcohol medium are situated and into which a fluid to be tested can be introduced, and means (11) for assessing effects of the fluid on one or more bacteriological actions selected from biological nitrification, denitrification, and phosphate uptake and release, wherein the bacteria have been immobilised by encapsulation using a freeze/thaw technique.

3. A detector (1) according to Claim 2 in which the means (11) for assessing effects of the fluid on one or more bacteriological actions selected from biological nitrification, denitrification, and phosphate uptake and release comprises means to which substances indicative of action of the bacteria are fed from the detector vessel (2).

4. A detector (1) according to Claim 2 or Claim 3 in which the means (11) for assessing effects of the fluid on one or more bacteriological actions selected from biological nitrification, denitrification, and phosphate uptake and release comprises means (11) arranged to assess substances within the detector vessel (2) which are indicative of action of the bacteria.

5. A detector (1) according to any of Claims 2 to 4 in which the bacteria are distributed amongst many small pieces of the medium.

6. A detector (1) according to any of Claims 2 to 5 in which the encapsulated bacteria occupy approximately 50% of the volume of the detector vessel (2).

7. A detector (1) according to any of Claims 2 to 6 which further comprises means (12) for monitoring pH level, temperature and/or oxygen level which are conditions essential to the well-being of the bacteria.

8. A detector according to Claim 7 in which a data processor (5) uses the output of the monitoring means to determine the percentage inhibition of one or more bacteriological actions selected from biological nitrification, denitrification, and phosphate uptake and release.

## Patentansprüche

1. Ein Verfahren zum Testen eines Fluids auf die Gegenwart von Substanzen, welche für ausgewählte Bakterien giftig sind, wobei Bakterien, welche durch Verkapselung in einem Polyvinylalkoholmedium immobil gehalten werden, dem Fluid ausgesetzt werden und die Effekte des Fluids auf ein oder mehrere bakteriologische Wirkungen ausgewählt aus biologischer Nitrifizierung, Denitrifizierung und Phosphataufnahme und -abgabe beurteilt werden, wobei die Bakterien unter Einsatz einer Gefrier-/Tautechnik durch Verkapselung immobilisiert worden sind.

2. Ein Detektor (1) zur Detektierung von Substanzen, welche für ausgewählte Bakterien giftig sind, umfassend ein Detektorgefäß (2), in welchem durch Verkapselung in einem Polyvinylalkoholmedium immobilisierte Bakterien enthalten sind und in welches ein zu testendes Fluid eingeführt werden kann, und eine Einrichtung (11) zum Beurteilen von Effekten des Fluids auf eine oder mehrere bakteriologische Wirkungen ausgewählt aus biologischer Nitrifizierung, Denitrifizierung und Phosphataufnahme und -abgabe, wobei die Bakterien unter Verwendung einer Gefrier-/Tautechnik durch Verkapselung immobilisiert worden sind.

3. Ein Detektor (1) gemäß Anspruch 2, in welchem die Einrichtung (11) zum Beurteilen von Effekten des Fluids auf eine oder mehrere bakteriologische Wirkungen ausgewählt aus biologischer Nitrifizierung, Denitrifizierung und Phosphataufnahme und -abgabe eine Einrichtung umfaßt, der Substanzen, die die Wirkung der Bakterien anzeigen, von dem Detektorgefäß (2) zugeführt werden.

4. Ein Detektor (1) gemäß Anspruch 2 oder Anspruch 3, in welchem die Einrichtung (11) zum Beurteilen von Effekten des Fluids auf eine oder mehrere bakteriologische Wirkungen ausgewählt aus biologischer Nitrifizierung, Denitrifizierung und Phosphataufnahme und -abgabe eine Einrichtung (11) umfaßt, die angeordnet ist, um Substanzen innerhalb des Detektorgefäßes (2) zu beurteilen, welche die Wirkung der Bakterien anzeigen.

5. Ein Detektor (1) gemäß einem der Ansprüche 2 bis 4, in welchem die Bakterien zwischen vielen kleinen Teilen des Mediums verteilt sind.

6. Ein Detektor (1) gemäß einem der Ansprüche 2 bis 5, in welchem die verkapselten Bakterien etwa 50% des Volumens des Detektorgefäßes (2) einnehmen.

7. Ein Detektor (1) gemäß einem der Ansprüche 2 bis 6, welcher desweiteren eine Einrichtung (12) zum Überwachen von pH-Wert, Temperatur und/oder Sauerstoffgehalt umfaßt, welche wesentliche Bedingungen für das Wohlergehen der Bakterien darstellen.

8. Ein Detektor gemäß Anspruch 7, in welchem ein Datenprozessor (5) den Ausgang der Überwachungseinrichtung nutzt, um den Inhibitionsprozentsatz von einer oder mehreren bakteriologischen Wirkungen ausgewählt aus biologischer Nitrifizierung, Denitrifizierung und Phosphataufnahme und -abgabe zu bestimmen.

## Revendications

1. Procédé permettant de tester la présence, dans un fluide, de substances toxiques pour des bactéries sélectionnées, dans lequel on met des bactéries, maintenues immobilisées par encapsulation dans un milieu constitué de poly(alcool vinylique), en présence du fluide et l'on évalue les effets du fluide sur une ou plusieurs activités bactériennes choisies parmi les processus biologiques de nitrification, de dénitrification et d'absorption et libération de phosphate, et pour lequel on a immobilisé les bactéries par encapsulation en ayant recours à une technique de congélation/décongélation.

2. Détecteur (1) destiné à détecter des substances toxiques pour des bactéries sélectionnées, comportant une cuve (2) de détecteur dans laquelle sont placées des bactéries, immobilisées par encapsulation dans un milieu constitué de poly(alcool vinylique), et dans laquelle on peut introduire un fluide à tester, et des moyens (11) permettant d'évaluer les effets du fluide sur une ou plusieurs activités bactériennes choisies parmi les processus biologiques de nitrification, de dénitrification et d'absorption et libération de phosphate, les bactéries ayant été immobilisées par encapsulation selon une technique de congélation/décongélation.

3. Détecteur (1) conforme à la revendication 2, dans lequel les moyens (11) permettant d'évaluer les effets du fluide sur une ou plusieurs activités bactériennes choisies parmi les processus biologiques de nitrification, de dénitrification et d'absorption et libération de phosphate comportent des moyens vers lesquels sont amenées, depuis la cuve (2) de détecteur, des substances indicatrices de l'activité des bactéries.

4. Détecteur (1) conforme à la revendication 2 ou 3, dans lequel les moyens (11) permettant d'évaluer les effets du fluide sur une ou plusieurs activités bactériennes choisies parmi les processus biologiques de nitrification, de dénitrification et d'absorption et libération de phosphate comportent des moyens (11) disposés de manière à ce qu'on puisse doser, à l'intérieur de la cuve (2) de détecteur, des substances indicatrices de l'activité des bactéries.

5. Détecteur (1) conforme à l'une des revendications 2 à 4, dans lequel les bactéries sont réparties entre de nombreux petits morceaux du milieu.

6. Détecteur (1) conforme à l'une des revendications 2 à 5, dans lequel les bactéries encapsulées occupent à peu près 50 % du volume de la cuve (2) de détecteur.

7. Détecteur (1) conforme à l'une des revendications 2 à 6, qui comporte en outre des moyens (12) permettant de surveiller les valeurs du pH, de la température et du taux d'oxygène, conditions essentielles pour le bien-être des bactéries.

8. Détecteur conforme à la revendication 7, dans lequel un appareil informatique (5) détermine, à partir des signaux de sortie des moyens de surveillance, le pourcentage d'inhibition d'une ou de plusieurs activités bactériennes choisies parmi les processus biologiques de nitrification, de dénitrification et d'absorption et libération de phosphate.
